# EUROPEAN PATENT APPLICATION

(11) **EP 3 915 573 A1**
(43) Date of publication of application: **01.12.2021**
(21) Application number: 20305554.6
(22) Date of filing: 27.05.2020
(51) Int. Cl.: A61K 38/38, A61P 7/08

(54) **TREATMENT OF COVID-19 PATIENTS**

(71) Applicant: Assistance Publique Hôpitaux de Paris, 75004 Paris (FR); Sorbonne Université, 75006 Paris (FR)
(72) Inventor: DEMERET, Sophie, 75014 PARIS (FR); SAHEB, Samir, 75010 PARIS (FR); WEISS, Nicolas, 92600 ASNIÈRES-SUR-SEINE (FR); ROHAUT, Benjamin, 75013 PARIS (FR); MAROIS, Clémence, 75013 PARIS (FR); LE GUENNEC, Loic, 75013 PARIS (FR); CAO, Albert, 94400 VITRY SUR SEINE (FR)
(74) Representative: Flesselles, Bruno F.G.

(57) **Abstract**

The present invention relates to use of plasma exchange therapy using only albumin as replacement for treating Covid-19 patients.

## Description

Critically ill patients infected by Severe Acute Respiratory Syndrome Coronavirus-2 (SARS-CoV-2) responsible for Coronavirus disease 2019 (Covid-19) may develop acute respiratory distress syndrome (ARDS) and multi-organ failure (1). Systemic host response to infection is thought to be highly involved, based on the observation of high plasma levels of pro-inflammatory cytokines, with interleukin (IL)-6 associated to severity and mortality in Covid-19 patients (2-4). Immune system activation and cytokine release syndrome (CRS) has been pointed out (5). This syndrome is not unique to Covid-19 and has been described in sepsis/septic shock for years with dramatic impact on inflammation, endothelial dysfunction, pathologic coagulation and finally on outcome (6). Targeting CRS has been recently proposed in the treatment of the most severe Covid-19 patients by using monoclonal antibodies directed against IL-6 or IL-1-Beta (7). Unfortunately, the best target is still unknown and these treatments are associated to a long-lasting immunosuppression.

Plasma exchange or plasma exchange therapy (PET or TPE) offers a unique opportunity to rapidly reduce several circulating cytokines present in severe cases of Covid-19. Indeed, TPE was found able to reduce pro-inflammatory cytokines in sepsis/septic shock even if, until now, no effect on outcome could be demonstrated (8-10). Recently, some authors reported the use of TPE in Covid-19 with however unconclusive results, using fresh frozen plasma (FFP) as replacement fluid (16, 17) Kesici et al (18) suggest that TPE applied with convalescent plasma (CP) should be considered as a therapeutic option in severe Covid-19 patients within the first week of symptom onset. Lin et al (19) reported TPE associated with continuous venovenous hemofiltration (CVVH). Ma et al (20) reported using blood purification therapies, including TPE and adsorption for treating Covid-19 patients.

Plasma exchange is the removal and retention of plasma, with return of the cellular components to the patients, in order to remove antibodies, toxins or abnormal proteins that are causing the clinical symptoms. The patient's blood goes through an apheresis machine, where the blood is centrifuged or filtered so as to isolate the plasma fraction, such plasma fraction is removed, and the red blood cells, white blood cells and platelets are reinfused to the patient, along with replacement fluid.

Typically, 30-40 mL/kg of plasma (1-1.5 plasma volumes) are removed at each procedure and replaced with Frozen Fresh Plasma, isotonic 4.5 or 5.0% human albumin solution or mixture of such. Frozen Fresh Plasma refers to the plasma portion of whole blood, centrifuged, separated, and frozen solid at -18 °C or colder within eight hours of collection, but may also be used to designate any transfused plasma product.

Blood plasma makes up about 55% of the body's total blood volume and its volume can be estimated by the formula: Estimated plasma volume (in liters) = 0.07 x weight (kg) x (1 - hematocrit) .

A one plasma volume (PV) exchange removes about 63% of intravascular constituents, 1.2 PV approximately around 70%, 1.5 PV approximately around 75%, and 2 PV remove around 85% of the constituents.

The inventors have determined that it is possible to treat Covid-19 patients, including those under mechanical ventilation by performing plasma exchange, and replacing the removed and discarded plasma by an isotonic solution containing from 4-6% albumin (preferably about 5% albumin, or 5% albumin).

The term "around" or "about" is meant to encompass variations of ±3%, or in preferred instances ±1%, as such variations are appropriate to perform the disclosed methods.

The invention thus relates to an isotonic solution containing from 4-6% albumin for use thereof for the treatment of a Covid-19 patients, as a plasma replacement in plasma exchange therapy. In the preferred embodiment, the isotonic solution contains 5% albumin. Such isotonic solution is known in the art and is commercialized for instance under the name of Viabelex by the Laboratoire Français des Biotechnologies (LFB, Les Ulis, France). It can be dosed at 4% (40 mg/mL) or at 20% (200 mg/mL). When the solution is highly dosed, it is diluted in saline to obtain the appropriate concentration. Such solution also contains sodium chloride and/or sodium caprylate and water so that it can be injected. The solution is isotonic for plasma, meaning that it has the same osmolarity or osmolality than plasma (280 to 295 mOsm/L).

In the context of the invention, it is preferred when the isotonic solution is the only solution reinjected to the patient as plasma replacement. In other words, the solution is the sole replacement fluid used in the plasma exchange therapy. This means that no frozen fresh plasma, or convalescent plasma is used.

The isotonic solution replaces plasma which has been discarded, so as to remove any factor that may be responsible for the cytokine storm observed in critically ill Covid-19 patients, in particular IL-6. A plasma volume exchange of 1 to 1.5, in particular of 1.2 is appropriate for using the isotonic solution.

The "cleaning" of the patient's plasma, leading to clinical improvement is generally not observed after one plasma exchange session. It is preferred to repeat such session, and when the isotonic solution is used multiple times. In particular, it is used from 3 to 5 times, once every 48 hours, particularly 4 times, once every 48 hours. However, the physician may decide to increase or decrease the number of uses or the duration between two uses, depending on the patient's clinical condition.

The isotonic solution can be used for any patient with Covid-19. However, since the plasma exchange procedure is a heavy clinical procedure, it is preferred when it is used for patients with severe Covid-19, in particular for patients in need for oxygen assistance, in particular patients with acute respiratory distress syndrome and/or multi-organ failure. The example shows that it can be used when the patient is under mechanical ventilation.

The invention also relates to a method for treating a patient with Covid-19, comprising performing plasma exchange for the patient, wherein an isotonic solution containing from 4 to 6% albumin is used as the sole replacement fluid for plasma before reinjection to the patient. The embodiments disclosed above are also applicable to this method.

The invention also relates to a method for preparing a composition for treating a Covid-19 patient, replacing removed plasma from a patient with Covid-19 with an isotonic solution containing from 4 to 6% albumin as the sole replacement fluid, wherein from 1 to 1.5 volumes of patient has been removed. Such composition is a medicament used for the treatment of the Covid-19 patient. The medicament thus consists in a composition which contains from 13.75% to 22% of plasma from the patient, from 33% to 41.25% of an isotonic solution containing from 4 to 6 % of albumin, about 4% of white cells, about 41% of blood cells and about 0.01% of platelets, such cells and platelets originating from the patient. This method is performed *ex vivo* and doesn't include the step of obtaining the patient's plasma or of reinjecting the composition to the patient.

These percentages correspond to the replacement of 1 to 1.5 plasma volumes by the isotonic solution, and considering that the blood plasma is about 55% of the blood volume, with is complemented by about 4% of white cells, about 41% of blood cells and traces of platelets. When a plasma volume of 1.2 is removed (corresponding to the removal of around 70% of the plasma content) and replaced with the isotonic solution, the composition contains 16.5% of plasma from the patient, 39.5% of the isotonic solution containing from 4 to 6 % of albumin, about 4% of white cells, about 41 % of blood cells and about 0.01 % of platelets.

Due to the fact that there is some patient's plasma remaining in the composition, even after addition of the isotonic solution, such composition contains antibodies or T lymphocytes against an SARS-CoV-2 antigen. Indeed, since the patient was infected with the SARS-CoV-2 virus, some immune response (humoral and cellular) is observable for the patient, thus leading to the presence of such antibodies or T lymphocytes.

The composition herein disclosed is obviously susceptible to be obtained by the method disclosed above. It is to be noted that the composition is generally administered to the patient as soon as it is produced in the apparatus performing the plasma removal. Indeed, the plasma exchange therapy requires some kind of extracorporal circulation, and the composition is not intended to be stored.

The invention also relates to the composition herein disclosed, for use thereof for the treatment of a Covid-19 patient. In particular, the plasma was isolated from the patient receiving the treatment. The composition is thus prepared from a patient's plasma and reinjected to this patient.

In summary, using the isotonic solution as disclosed as the sole replacement fluid in TPE offers a lots of benefits:
- Such solutions are widely available and more easily than fresh frozen plasma
- These solutions are more safe than fresh frozen plasma, in view of their manufacture processes that lower the risk of contamination, where fresh frozen plasma or convalescent plasma that may contain pathogens
- These solutions reduce the anaphylactic risk (or other risks) that is sometimes observed with plasma transfusion (15).

### FIGURE

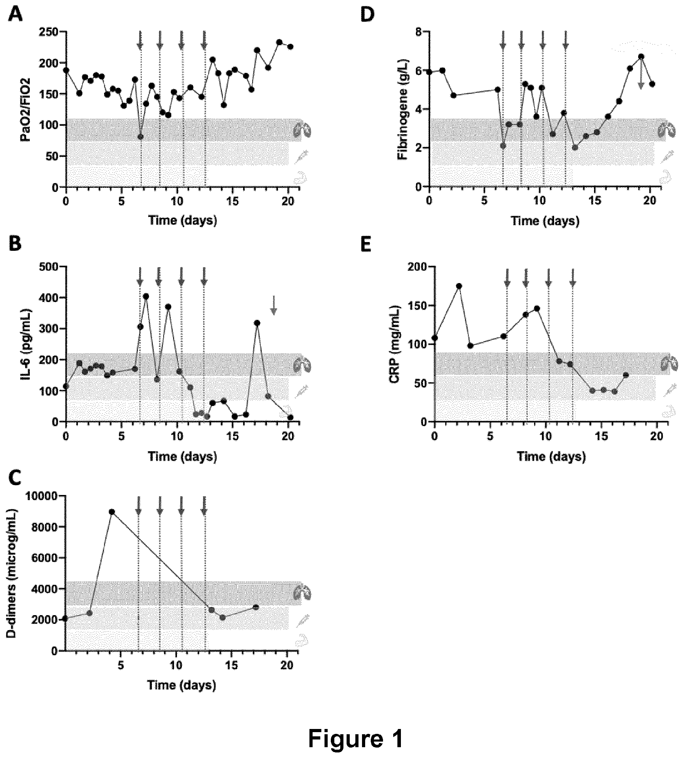

Figure 1: evolution of the PaO2/FiO2 (A), IL-6 (B), D-dimers (C), fibrinogene (D), and CRP € values over time following TEP"(represented by the four top arrows with dotted lines). A *Pseudomonas aeruginosa* ventilator-associated pneumoniae is represented by the arrow on the left (B and D panels). The mechanical ventilation duration is represented by the lung icon, the administration of sedative is represented by the syringe icon and the administration of neuromuscular blockers is represented by the biceps icon.

### EXAMPLES

### Example 1. Rapid resolution of ARDS secondary to SARS-CoV-2 infection by TPE

A 74-year-old man was admitted to ICU for acute respiratory distress that rapidly worsened over 3 days after having been in close contact with his son in-law presenting with signs highly suggestive of Covid-19. His past medical history was remarkable for hypertension treated with an association of calcium-channel and beta-blockers, a recent diagnosis of diabetes mellitus needing only dietary measures, and overweight (body mass index of 28,8 kg/m²). Upon ICU admission, respiratory rate was at 32 per minute and oxygen blood saturation (SatO₂) was at 81% in room air. Despite 15 liters per minute of oxygen through a facemask, SatO₂ was only at 91%. Orotracheal intubation to put the patient on mechanical ventilation was decided. Sedatives and neuromuscular blockers (NMB) were started simultaneously with lung protective ventilation. Biological values on admission are shown in Table 1.

**Table 1. Biological values on patient's admission Reference ranges are affected by many variables, including the patient population and the laboratory methods used. The ranges used at Pitié-Salpêtrière Hospital are for adults who are not pregnant and do not have medical conditions that could affect the results. They may therefore not be appropriate for all patients.**

| Variable | Patient results | Reference Range |
|---|---|---|
| Tracheal Aspirate | | |
| RT-PCR for SARS-CoV-2 | Positive | |
| Blood | | |
| Hemoglobin (g/dl) | 15.0 | 13-17.5 |
| Platelets (per mm³) | 180,000 | 150,000-400,000 |
| White-cell count (per mm³) | 9290 | 4000-10,000 |
| Differential count (per mm³) | | |
| Total neutrophils | 7170 | 2000-7500 |
| Total lymphocytes | 1680 | 1500-4000 |
| Total monocytes | 400 | 150-1000 |
| Procalcitonine (µg/l) | 0.71 | < 0.1 |
| High-sensitivity C-reactive protein (mg/liter) | 75 | < 5 |
| Fibrinogen (g/liter) | 5.1 | 2-4 |
| D-Dimers (ng/ml) | 890 | < 500 |
| NT-pro-BNP (ng/l) | 72.6 | < 300 |
| Interleukine-6 (pg/ml) | 114.2 | 0-6.5 |
| Serologic testing for human immunodeficiency virus | Negative | |
| Serologic testing for hepatitis b & c | Negative | |

RT-PCR on nasopharyngeal swab confirmed SARS-CoV-2 infection. Chest X-ray revealed bilateral interstitial infiltrates suggestive of Covid-19 related ARDS. Empiric antibiotic therapy with IV cefotaxime was introduced on arrival, switched to piperacillin and tazobactam for seven days because of distal protected aspirate positive for oral flora.

Despite this treatment, the patient respiratory status worsened over the 7 first days with a PaO₂/FiO₂ ratio at 79 under PEEP at 14 cmH₂O (pulmonary dynamic compliance at 27,6 mL/cmH₂0) at day 7. The levels of CRP (110 mg/L, normal range < 5), fibrinogen (5.0 g/L, normal range 2-4) and IL-6 (306 pg/mL, normal range < 6,5) were increased.

TPE indication was retained and the patient underwent 4 consecutive sessions every 48 hours (Spectra Optia, TerumoBCT, Lakewood, CO, USA), with 5% albumin as the sole replacement solution, exchanging 1.2 plasma volumes with a blood flow of 60 (40-70) mL/min for a duration of 110 (93-120) minutes. Anticoagulation was achieved by regional citrate infusion (1/12). Evolution of PaO₂/FiO₂ ratio, CRP, fibrinogen and IL-6 levels are shown in Figure 1. The last TPE session occurred on day 13.

### Results

The patient respiratory status rapidly improved. He could be weaned from NMB at day 12, from sedatives on day 20 and finally from mechanical ventilation on day 21. Whereas PaO₂/FiO₂ ratio increased, IL-6 serum levels decreased under TPE treatment after initial rebounds, as expected with a protein of low molecular weight (24 kDa), short half-life, and broad distribution space (11). The patient's status remained stable as the IL-6 levels (Figure 1).

In this patient, TPE was used as a unique strategy to mitigate CRS associated to Covid-19 without any *a priori* of the most important cytokine implicated in the syndrome. Our patient spectacularly improved under treatment, as compared to the dramatical outcomes described in Covid-19 patients admitted to ICU (13), and had no relapse after TPE discontinuation of both his respiratory status and his plasmatic IL-6 levels (except for a peak due to *Pseudomonas aeruginosa* ventilator-associated penumoniae). TPE has the advantage over monoclonal antibodies targeting pro-inflammatory cytokines of his transient effect on immune system precluding any long-lasting immunosuppression.

In addition, the protocol that was used here, with 5% albumin as sole replacement fluid, offers major benefits in terms of availability and security as compared to replacement with fresh frozen plasma or convalescent plasma that has been proposed for treatment of critically ill Covid-19 patients (14), with inherent anaphylactic risk.

### REFERENCES

1. Huang C, Wang Y, Li X, et al. Clinical features of patients infected with 2019 novel coronavirus in Wuhan, China. Lancet 2020; 395: 497-506.
2. Zhou F, Yu T, Du R, et al. Clinical course and risk factors for mortality of adult inpatients with COVID-19 in Wuhan, China: a retrospective cohort study. The Lancet 2020;395(10229):1054-62.
3. Ruan Q, Yang K, Wang W, Jiang L, Song J. Clinical predictors of mortality due to COVID-19 based on an analysis of data of 150 patients from Wuhan, China. Intensive Care Med 2020;46(5):846-8.
4. Wang C, Fei D, Li X, Zhao M, Yu K. IL-6 may be a good biomarker for earlier detection of COVID-19 progression. Intensive Care Med. 2020;1-2. doi:10.1007/s00134-020-06065-8
5. Mehta P, McAuley DF, Brown M, Sanchez E, Tattersall RS, Manson JJ. COVID-19: consider cytokine storm syndromes and immunosuppression. The Lancet 2020; 395(10229):1033-4.
6. Keith P, Day M, Perkins L, Moyer L, Hewitt K, Wells A. A novel treatment approach to the novel coronavirus: an argument for the use of therapeutic plasma exchange for fulminant COVID-19. Crit Care 2020;24(1):128, s13054-020-2836-4.
7. Sanders JM, Monogue ML, Jodlowski TZ, Cutrell JB. Pharmacologic Treatments for Coronavirus Disease 2019 (COVID-19): A Review. JAMA 2020;323(18):1824-36.
8. Rimmer E, Houston BL, Kumar A, et al. The efficacy and safety of plasma exchange in patients with sepsis and septic shock: a systematic review and meta-analysis. Crit Care 2014;18(6):699
9. Reeves JH: A review of plasma exchange in sepsis. Blood Purif 2002; 20:282-288 [PubMed: 11867876]
10. Knaup H, Stahl K, Schmidt BMW, et al. Early therapeutic plasma exchange in septic shock: a prospective open-label nonrandomized pilot study focusing on safety, hemodynamics, vascular barrier function, and biologic markers. Crit Care 2018;22(1):285.
11. Reverberi R, Reverberi L. Removal kinetics of therapeutic apheresis. Blood Transfus 2007;5(3):164-74.
12. Shariatmadar S, Nassiri M, Vincek V. Effect of plasma exchange on cytokines measured by multianalyte bead array in thrombotic thrombocytopenic purpura. Am J Hematol. 2005;79(2):83-88. doi:10.1002/ajh.20342
13. Richardson S, Hirsch JS, Narasimhan M, et al. Presenting characteristics, comorbidities, and outcomes among 5700 patients hospitalized with COVID-19 in the New York City area. JAMA Published online April 22. 2020. 10.1001/jama.2020.6775.
14. Kesici S, Yavuz S, Bayrakci B. Get rid of the bad first: Therapeutic plasma exchange with convalescent plasma for severe COVID-19. Proc Natl Acad Sci USA 2020;202006691.
15. Pandey and Vyas, Transfusion. 2012 May; 52(Suppl 1): 65S-79S
16. Adeli et al, Pol Arch Intern Med, 2020 May 7
17. Shi et al International Journal of Antimicrobial Agents (2020), doi: doi.org/10.1016/j.ijantimicag.2020.105974)
18. Kesici et al Proc Natl Acad Sci USA. 2020 May 12;202006691
19. Lin et al Journal of the Formosan Medical Association, doi.org/10.1016/j.jfma.2020.04.023
20. Ma et al (Clinical Immunology 214 (2020) 108408)

## Claims

1. Isotonic solution containing from 4-6% albumin for use thereof for the treatment of a Covid-19 patient, as the sole replacement fluid for plasma replacement in plasma exchange therapy.

2. Isotonic solution for use according to claim 1, which contains 5% albumin.

3. Isotonic solution for use according to claim 1 or 2, wherein from 1 to 1.5 volumes of plasma is exchanged.

4. Isotonic solution for use according to any one of claims 1 to 3, wherein it is used from 3 to 5 times every 48 hours.

5. Isotonic solution for use according to any one of claims 1 to 4, wherein the patient is under mechanical ventilation.

6. A method for preparing a composition for treating a COVID-19 patient, replacing removed plasma from a patient with COVID-19 with an isotonic solution containing from 4 to 6% albumin as the sole replacement fluid, wherein from 1 to 1.5 volumes of patient has been removed.

7. An composition for treating COVID-19 solution, containing from 13.75 to 22% plasma, from 33 to 41.25% of an isotonic solution containing from 4 to 6 % of albumin, about 4% of white cells, about 41% of blood cells and about 0.01% of platelets,

8. The composition of claim 7, which contains antibodies or T lymphocytes against an SARS-CoV-2 antigen.

9. The composition of claim 7 or 8, which is susceptible to be obtained by the method of claim 6.

10. The composition of any one of claims 7 to 8 for use thereof for the treatment of a COVID-19 patient.

11. The composition for use of claim 10, wherein the plasma was isolated from the patient receiving the treatment.
